# EUROPEAN PATENT APPLICATION

(11) **EP 1 785 488 A1**
(43) Date of publication of application: **16.05.2007**
(21) Application number: 05110516.1
(22) Date of filing: 09.11.2005
(51) Int. Cl.: C12N 15/861, A61K 48/00

(54) **Adenoviral vectors with two separate expression cassettes**

(71) Applicant: Crucell Holland B.V., 2333 CN Leiden (NL)
(72) Inventor: Vogels, Ronald, 3461 HW Linschoten (NL); Zuijdgeest, David A.T.M., 2565 VH Den Haag (NL)
(74) Representative: Klein, Bart

(57) **Abstract**

The invention relates to recombinant replication-defective adenoviral vectors comprising two separate expression cassettes, each cassette enabling the high expression of a protein encoded by a nucleic acid of interest. Each cassette comprises a promoter and a polyadenylation signal. An important aspect of the recombinant adenoviruses according to the invention is that they remain stable upon numerous passages in cell culture. Assays to monitor the stability of such adenoviruses over multiple passages are provided.

## Description

### FIELD OF THE INVENTION

The invention relates to the field of medicine, more in particular to the field of biotechnology, wherein gene delivery vehicles based on replication-defective recombinant adenoviruses are used in medicaments.

### BACKGROUND OF THE INVENTION

Adenoviruses are presently widely used and tested in medical applications. Typically, recombinant adenoviruses are made replication-defective by deletion of one or more genes that are essential for replication from the viral backbone, rendering the virus incapable of replicating in a host cell. Generally, recombinant adenoviruses are made replication-defective by removal of most if not all of the early region-1 (E1) part of the viral genome. Such viruses can be produced in so-called packaging cell systems, wherein a cell is used that has the E1 of an adenovirus stably incorporated into its cellular genome, thereby providing the missing, but for replication essential, E1 function to the virus replication machinery. Examples of packaging cells that are used in the art are the human kidney-derived 293 cells, 911 cells, and the human retinoblast-derived PER.C6^{®} cells.

It was recognized in the field of adenoviral vectors that many individuals around the world become infected with many of the generally studied and produced recombinant adenovirus serotypes, exemplified by Ad2 and Ad5. It was found that only a relative small percentage of individuals were infected with a number of serotypes, that, for that reasons were referred to as 'rare' serotypes. Examples of such rare serotypes are Ad11, Ad34, Ad35, Ad48, Ad49 and Ad50. Recombinant vectors based on such viruses encounter titers of neutralizing antibodies only in a small percentage of human beings around the world, thereby making such vectors useful in the application of vaccines or gene therapy medicaments, in which the presence of neutralizing antibodies is unwanted. Also in prime-boost set ups in vaccine-related therapies it is preferred to use recombinant adenoviral vectors that do not encounter neutralizing antibodies in the host, for instance due to a previous infection, or a preceding prime-vaccination.

In the field of adenoviral technology, one has always looked for ways to incorporate a large variety of transgenes or nucleic acids encoding antigens. At a certain stage, it was realized that the capacity of the adenoviral vector to become packaged in a packaging system, was limited. The size of the genome that can actually be packaged is limited. It is generally believed that for instance for Ad5, the genome cannot be larger than 105% of its wild type size. Also in the low-neutralized vector based on Ad35, the maximum size of the genome is around 105% to 106% (the Ad35 gene encoding the fiber is smaller than the Ad5 fiber gene).

Already in the early days of recombinant adenoviral technology, one realized that certain parts of the viral genome could be left out, without affecting the virus, the replication and the packaging thereof. One of the main regions that appeared to be redundant for many of the applications in gene therapy and/or vaccination, was the early region 3 (E3) in the viral backbone. One of the major accomplishments in the field of adenoviral technology was the manufacturing of a recombinant vector in which a transgene was incorporated, which transgene was expressed from the viral backbone, after infection of the virus in cells (be it in vitro or in vivo). Typically, the transgene was placed in an expression cassette comprising a promoter, followed by the transgene and terminated by a poly-adenylation (pA) signal. The promoter was either adenoviral derived, such as the Major Late Promoter, or the E1 promoter, that originally was present to express the E1 proteins. Also heterologous promoters were applied, such as the Rous Sarcoma Virus (RSV) promoter or the Cytomegalovirus (CMV) promoter, the latter being preferred because of its high expression levels. The expression cassettes typically comprise multiple cloning sites enabling one to clone different kinds of transgenes, or antigen-encoding nucleic acids. Also, the pA signal is generally heterologous to the backbone vector, where the SV40 and the Bovine Growth Hormone (BGH) pA signals are preferred.

The deletion of the E3 region enabled one to clone larger nucleic acids of interest into the viral genome, as it simply provided more space and thus, the possibility to get packaged. This system was further developed into settings where almost all adenoviral DNA was removed, in so-called minimal vectors, but since many of the essential components of the adenovirus could not be stably integrated into the genome of packaging cells (generally due to toxicity issues), such minimal vectors require the co-infection of helper viruses, making those systems not highly suitable for large-scale production, which is required when one intends to produce vaccines or gene therapy products for worldwide use.

It was then realized that for certain particular applications, it would be helpful to incorporate multiple antigenic determinants, as some diseases are not treated by just one antigenic response, or one gene of interest in gene therapy. For instance, infectious diseases such as malaria are characterized in that the parasite that causes the infection, proceeds through multiple stages in its life-cycle, being present in different compartments within the infected host during those different stages. It is known that different antigens are expressed during different phases of that life cycle. It would therefore be beneficial to attack the parasite not only when it is present in for instance the liver stage, but also when it is floating around in the bloodstream during the pre-liver stage. The vaccination against such a parasite would benefit from a vaccine comprising multiple antigens. Moreover, some antigens only induce an antibody response, while for a proper treatment of the disease it would also be beneficial to have a cellular response and active T cells are required to combat the disease sufficiently. When different antigens from one organism induce different immune responses, it is clear that one would desire to apply multiple antigens during vaccination.

One way of achieving this is by the use of multiple vectors, each comprising a separate antigen. However, this requires the production of those multiple vectors, increasing the costs and research and labor intensiveness in a dramatic way, each time a new vector needs to be designed, build and produced for clinical application. Yet another way of solving this issue is the use of fusion products, in which one antigen is fused to another antigen or antigenic part of a protein, and expressed from the same promoter. However, as indicated above, this is limited by the available space in the viral vector. Also, not all antigens are suitable for expression as a fusion protein, due to folding problems and presentation and processing to, and in the immune system of the host.

It was realized that the E1 region as well as the E3 region of the adenovirus could be used to insert separate expression cassettes. This would enable one to have two separate nucleic acids of interest (transgenes, antigens, etc.) expressed from a single virus. Such vectors were made in the art (EP 1308517; US 6,544,780; Danthinne 1999 and 2001; Lipinski et al. 2001; Martin-Duque et al. 2004; Tai et al. 2003). However, most of the attempts to make such vectors proved unsuccessful because the adenoviral genome is prone to recombination. It is required for an application in the medical field that such viruses are stable over numerous passages, such that the adenovirus can be produced to an extent in which enough material is available to be used in the preparation of a medicament. The application of separate expression cassettes that have overlapping sequences make that the viruses thus produced are unstable. Double homologous recombination events cause deletions in the vector and typically a loss of one or two of the expression cassettes, including the nucleic acid(s) of interest, after a few passages in packaging cells. The problem that is to be solved is therefore how to provide a single adenovirus-based recombinant replication-defective viral vector that is suitable for application in mammals, preferably humans, which encounters low-neutralizing activity in the general human population, and which ensures the stable expression of two separate nucleic acids of interest, without a loss of stability in a required number of subsequent passages in packaging cells. The preferred number of passages over which the adenovirus should remain stable is at least eight. This would enable one to make the adenovirus in enough quantities to produce a workable medicament.

### BRIEF DESCRIPTION OF THE DRAWINGS

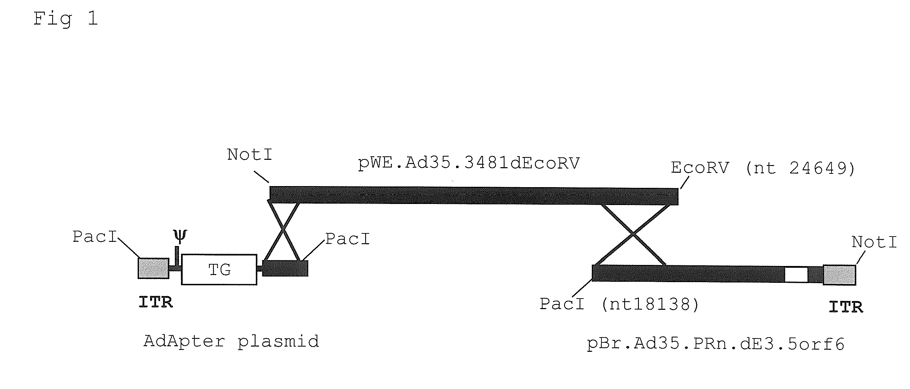

Figure 1: Schematic picture showing the three-plasmid system used to generate recombinant Ad35-based viruses through co-transfection on PER.C6 cells.

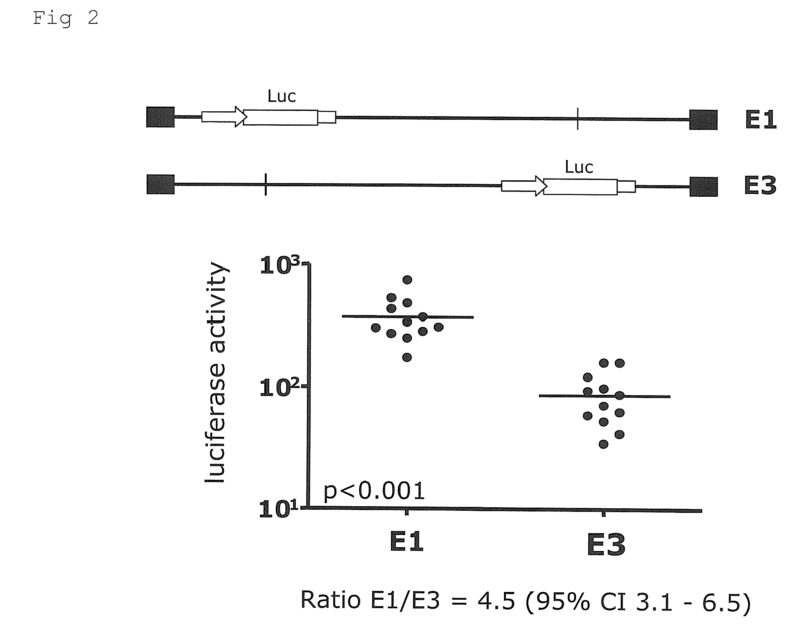

Figure 2: Comparison of expression levels mediated by the CMV promoter driving a luciferase gene in either the E1 region or the E3 region in recombinant Ad35 viruses. The used (single-insert) viruses are schematically indicated; the vertical line indicates a deletion (either E1 or E3 region). Both viruses are deleted for E1 and most of the E3 functions. The expression cassettes contain the human CMV promoter, luciferase gene and the SV40pa. The experiment was conducted twice in triplicate with 2 independent crude lysates of each virus.

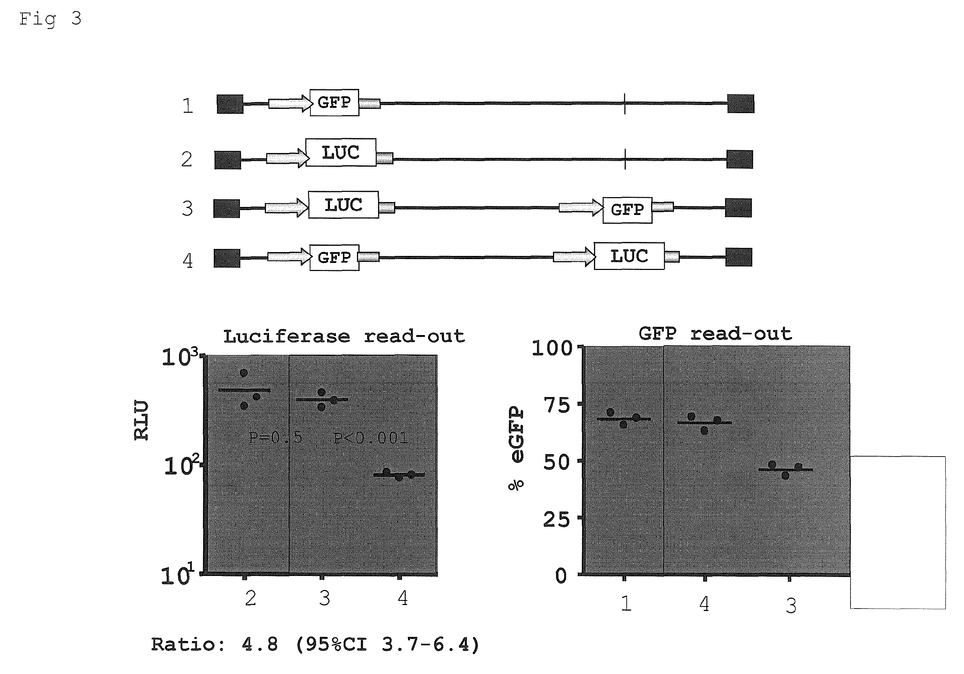

Figure 3: Analysis of expression levels of single and double insert viruses. The viruses are schematically indicated. The left graph presents the results of luciferase measurement (relative light units) and the right graph presents the results of the GFP measurement (percentage of eGFP-positive cells). The numbers of the viruses in the schematic drawing correspond to the numbers on the x-axis of the graphs.

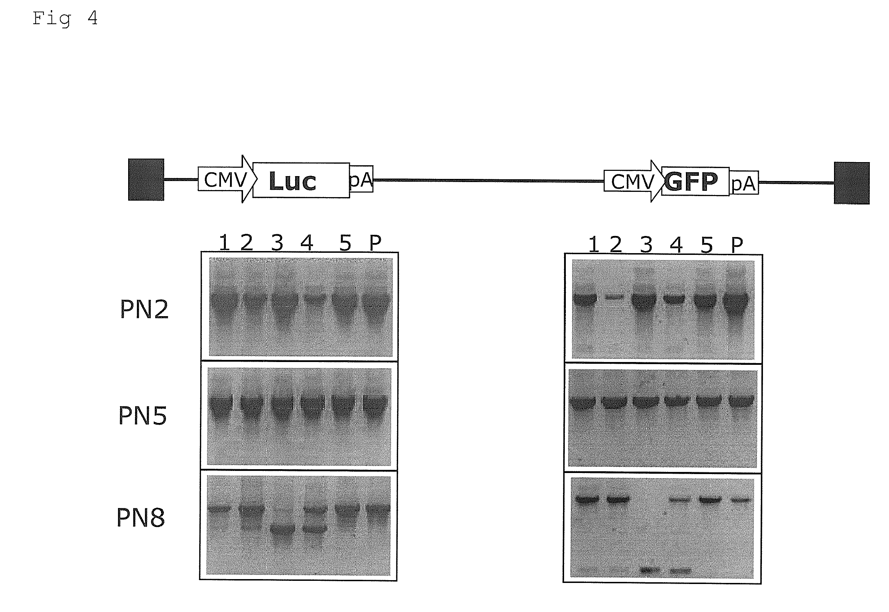

Figure 4: Stability analysis of E1/E3 double insert virus harboring the Luciferase transgene in E1 and the eGFP transgene in E3. DNA was isolated from viruses at PN2, PN5 and PN8 and analyzed by PCR for integrity of the expression cassettes.

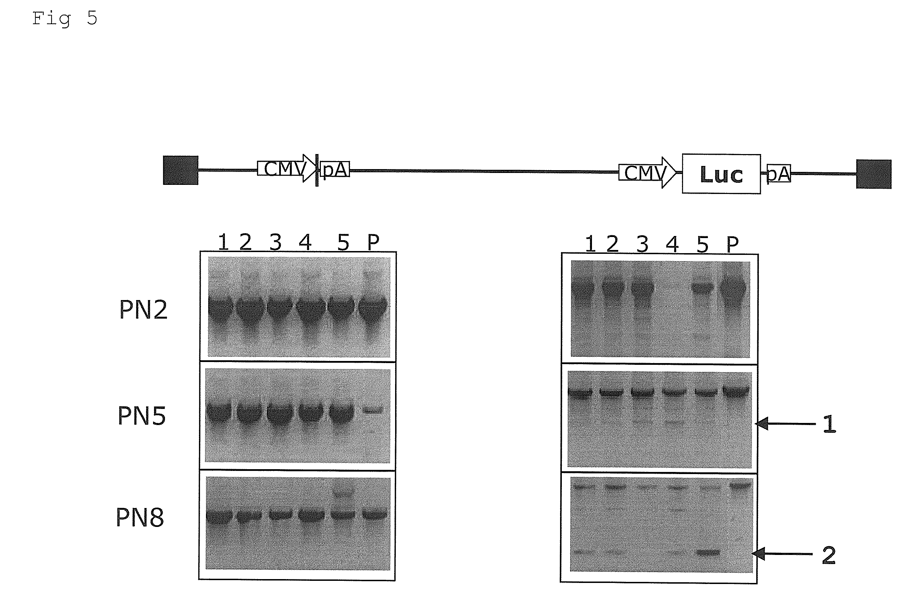

Figure 5: Stability analysis of E1/E3 double insert viruses: an empty CMVpA cassette in E1 and a CMV-Luciferase cassette in E3. Five independently grown virus preps were tested (1-5). P indicates the plasmid control which is the plasmid containing the region to be analyzed (E1 or E3) and which was used to generate the virus. PCR fragments indicating deletions in the transgene region that were analyzed by sequencing are indicated with an arrow.

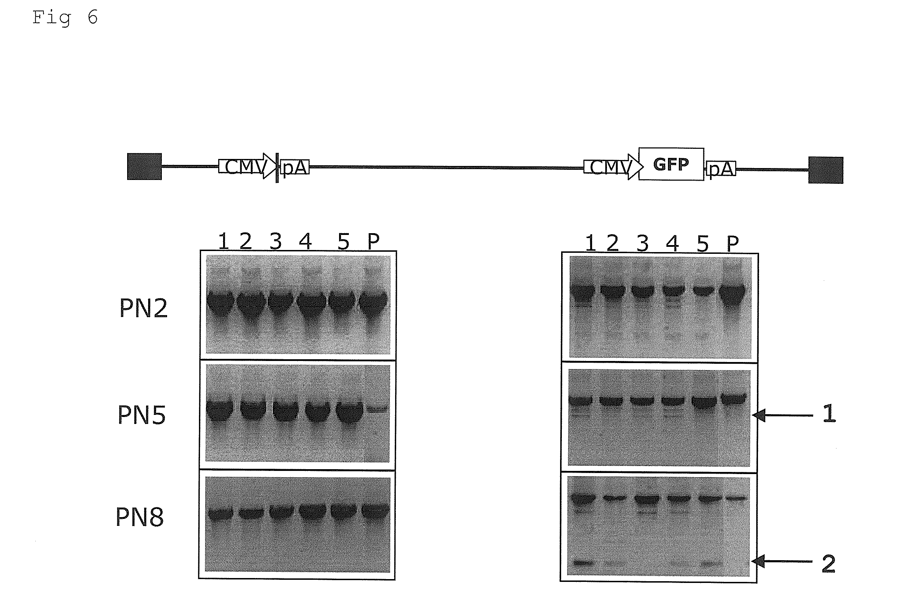

Figure 6: Stability analysis E1/E3 double insert viruses: an empty CMVpA cassette in E1 and a CMV-eGFP cassette in E3. Five independently grown virus preps were tested (1-5). P indicates the plasmid control which is the plasmid containing the region to be analyzed (E1 or E3) and which was used to generate the virus. PCR fragments indicating deletions in the transgene region that were also sequenced are indicated with an arrow.

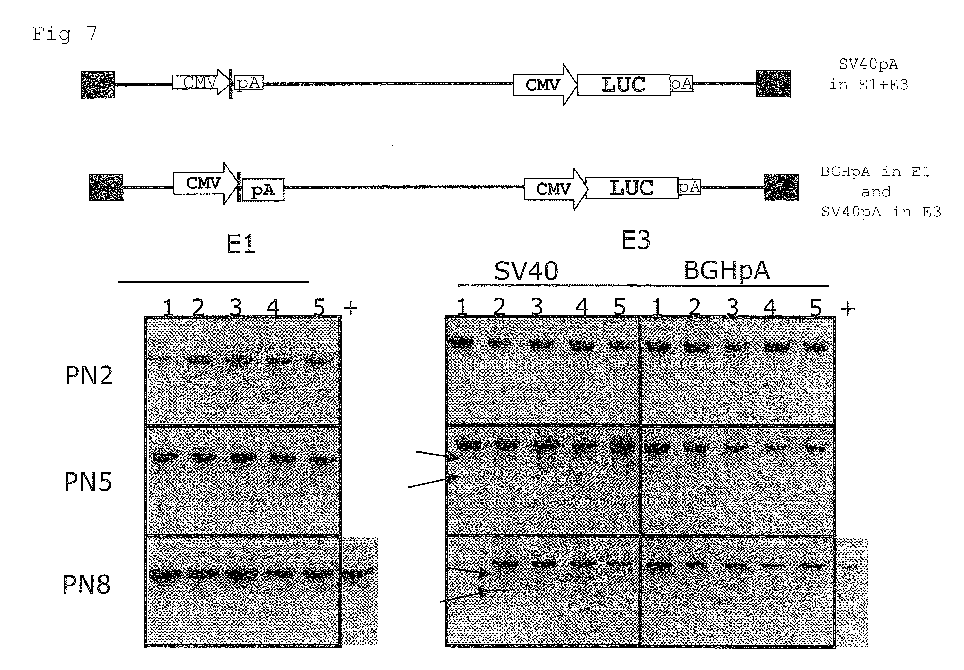

Figure 7: Stable double insert viruses with different pA sequences in the expression cassettes in E1 and E3 region: Luciferase transgene. Transgene stability of viruses with two SV40pA sequences or one SV40pA and a BGHpA sequence was analyzed by PCR at different passage numbers. The arrows indicate deletion fragments in the viruses with two SV40 pA sequences. Five independently grown virus preps were tested (1-5). P indicates the plasmid control which is the plasmid containing the region to be analyzed (E1 or E3) and which was used to generate the virus.

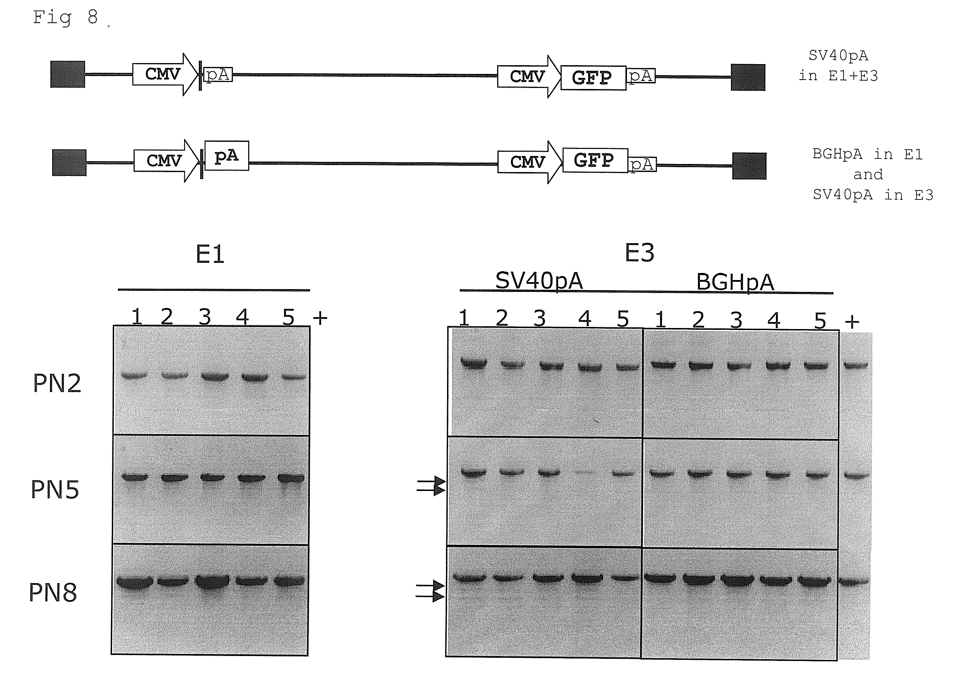

Figure 8: Stable double insert viruses with different pA sequences in the expression cassettes in E1 and E3 region: eGFP transgene. Transgene stability of viruses with two SV40pA sequences or one SV40pA and a BGHpA sequence was analyzed by PCR at different passage numbers. The arrows indicate deletion fragments in the viruses with two SV40 pA sequences. Five independently grown virus preps were tested (1-5). P indicates the plasmid control which is the plasmid containing the region to be analyzed (E1 or E3) and which was used to generate the virus.

### SUMMARY OF THE INVENTION

The invention relates to recombinant replication-defective adenoviruses comprising two separate expression cassettes for the expression of two separate nucleic acids of interest. The adenoviruses according to the invention are stable and do not recombine due to homologous recombination between any overlapping sequences present in the two separate expression cassettes.

### DETAILED DESCRIPTION

The inventors of the present invention herewith provide a solution to most of the problems outlined above. The invention relates to a single adenoviral vector, preferably based on human adenovirus serotype Ad35, which comprises two separate expression cassettes, enabling the insertion of two nucleic acids of interest. Both nucleic acids are well expressed from the viral backbone. Most importantly, the invention relates to a vector that cannot only be produced, despite the risk of recombination, but which is stable over multiple passages, preferably eight, thereby enabling the production of this vector to a large scale, without having the risk of finally ending up with an empty vector, from which the cassettes have been removed or re-shuffled. 'Stable' as used herein means that there are no detectable rearrangements of the adenoviral genome in the specific expression cassettes (preferably cloned in E1 and E3), by using an agarose gel analysis as described herein. The invention discloses how the skilled person can monitor whether an adenovirus so produced remains stable over numerous passages. PCR primers and PCR set-ups are provided that enables one to perform PCR reactions and analyze whether the E1 and E3 domains (comprising the separate inserts) remain stable. If not, one can identify smaller fragments of these regions over time, and over multiple passages in agarose gel analyzes, as described below.

The present invention relates to a recombinant replication-defective adenovirus having an adenoviral genome comprising two separate expression cassettes for the expression of two nucleic acids of interest, said expression cassettes each comprising a promoter and a poly-adenylation signal sequence. In a preferred embodiment, the recombinant adenovirus according to the present invention comprises an adenoviral genome that is stable for at least eight passages. Passages are defined as disclosed in the examples herein. This number of passages ensures a period that is long enough for the production of clinical lots for the treatment of individuals, preferably mammals, more preferably humans. The examples describe methods and means for the determination whether a produced adenovirus with two separate expression cassettes is indeed stable during the subsequent passages. PCR primers and PCR set-ups are provided, such that the skilled person is able to repeat the stability assays on viruses produced.

In another preferred embodiment, the recombinant adenovirus according to the invention comprises a genome, wherein said genome comprises a deletion in the E1 region and a deletion in the E3 region. Generally, the E1 deletion is a deletion of the functional part(s) of the E1 region, through which the adenovirus has become replication-defective. Preferably, all of the protein-encoding regions of the E1 domain are deleted, thereby requiring the expression of the E1A and E1B proteins from the packaging cell in which the replication-defective adenovirus can be produced and can replicate. Clearly, replication-defective means that the adenovirus cannot replicate in non-packaging cells. Packaging systems for replication and production of E1-deleted adenoviruses are well known in the art. The E3 deletion ensures the availability of more space, such that a second expression cassette can be inserted in that particular region, while the first expression cassette is present in the E1 region. Thus, the invention relates to a recombinant adenovirus according to the invention, wherein one of said two expression cassettes is cloned into the E1 deletion, and the other expression cassette is cloned into the E3 deletion. In yet another preferred embodiment, the recombinant adenovirus according to the invention, is based on an adenovirus serotype selected from Ad11, Ad34, Ad35, Ad48, Ad49 and Ad50. More preferred is a recombinant adenovirus, wherein said adenovirus is based on Ad35.

The invention also relates to a recombinant adenovirus according to the invention, wherein said separate expression cassettes comprise the same promoter. Although overlapping sequences within the two cassettes increase the risk of homologous recombination, it is still preferred to have high expression of both nucleic acids of interest, respectively expressed by the separate expression cassettes, while maintaining the stability of the genome and thus of the virus. Moreover, when overlapping sequences are used, more in particular, the same (strong) promoter is used, one takes the risk of squelching or promoter interference. It was surprisingly found that when using two CMV promoters in one adenoviral vector, one did not encounter promoter interference and one was able to retain stability for at least eight passages in cell culture. Therefore, in a highly preferred embodiment, the recombinant adenovirus according to the invention comprises two expression cassettes in its genome, each cassette comprising the CMV promoter as the promoter driving the expression of the nucleic acids of interest. The promoters are typically cloned upstream of the reading frame of the nucleic acid of interest and operably linked to said nucleic acids of interest. To increase the stability, it was found that limiting the overlapping regions between the two separate expression cassettes to a single region (preferably the promoter), the other possible overlapping region (the pA signal) should preferably be different. Therefore, the present invention, in a preferred embodiment, relates to a recombinant adenovirus according to the invention, wherein said separate expression cassettes comprise two different poly-adenylation signal sequences. Preferred examples of pA signals are the SV40 pA and the Bovine Growth Hormone (BGH) pA.

The invention further relates to a recombinant adenovirus according to the invention for use as a medicament. Such medicaments can be used for the treatment, prophylaxis or diagnosis of infectious diseases. They can also be used in other settings, such as gene therapy or tumor vaccination. Depending on the disease to be treated, prevented or diagnosed, the antigens are selected and can be co-expressed from a single adenoviral vector. Preferred examples of antigens that can be cloned into the adenoviral vector according to the present invention are antigens from viruses such as HIV (Rev, Pol, Nef, Gag, Env, Tat, mutant derivatives of Tat, such as Tat-Δ31-45, T- and B-cell epitopes of gp120, chimeric derivatives of HIV-1 Env and gp120, such as a fusion between gp120 and CD4, a truncated or modified HIV-1 Env, such as gp140 or derivatives of HIV-1 Env and/or gp140), hepatitis B surface antigen, rotavirus antigens, HCV, HSV, ebola virus, Marburg virus, and influenza virus. Thus, the viral pathogens, from which the viral antigens are derived, include, but are not limited to: Orthomyxoviruses, such as influenza virus; Retroviruses, such as RSV, HTLV-1, and HTLV-II, Herpesviruses such as EBV; CMV or herpes simplex virus; Lentiviruses, such as HIV-1 and HIV-2; Rhabdoviruses, such as rabies virus; Picornaviruses, such as Poliovirus; Poxviruses, such as vaccinia virus; Rotavirus; and Parvoviruses, such as Adeno-Associated Viruses (AAV). Examples of bacterial pathogens, from which bacterial antigens may be derived, include but are not limited to, *Mycobacterium spp., Helicobacter pylori, Salmonella spp., Shigella spp., E. coli, Rickettsia spp., Listeria spp., Legionella pneumoniae, Fansicella spp., Pseudomonas spp., Vibrio spp.,* and *Borellia burgdorferi.* Examples of Tuberculosis antigens that may be cloned into the expression cassettes are: Ag85A (MPT44), Ag85B (MPT59), Ag85C (MPT45), TB10.4 (CFP7), ESAT-6, CFP7A, CFP7B, CFP8A, CFP8B, CFP9, CFP10, CFP10A, CFP11, CFP16, CFP17, CFP19, CFP19A, CFP19B, CFP20, CFP21, CFP22, CFP22A, CFP23, CFP23A, CFP23B, CFP25, CFP25A, CFP26 (MPT51) CFP27, CFP28, CFP29, CFP30A, CFP30B, CWP32, CFP50, MPT63, MTC28, LHP, MPB59, MPB64, MPT64, TB15, TB18, TB21, TB33, TB38, TB54, TB12.5, TB20.6, TB40.8, TB10C, TB15A, TB17, TB24, TB27B, TB13A, TB64, TB11B, TB16, TB16A, TB32, TB32A, TB51, TB14, TB27, HBHA, GroEL, GroES (WO 95/01441, WO 98/44119, US 6,596,281, US 6,641,814, WO 99/04005, WO 00/21983, WO 99/24577), and the antigens disclosed in WO 92/14823, WO 95/14713, WO 96/37219, US 5,955,077, US 6,599,510, WO 98/31388, US 2002/0150592, WO 01/04151, WO 01/70991, WO 01/79274, WO 2004/006952, WO 97/09428, WO 97/09429, WO 98/16645, WO 98/16646, WO 98/53075, WO 98/53076, WO 99/42076, WO 99/42118, WO 99/51748, WO 00/39301, WO 00/55194, WO 01/23421, WO 01/24820, WO 01/25401, WO 01/62893, WO 01/98460, WO 02/098360, WO 03/070187, US 6,290,969, US 6,338,852, US 6,350,456, US 6,458,366, US 6,465,633, US 6,544,522, US 6,555,653, US 6,592,877, US 6,613,881, US 6,627,198. Examples of protective antigens of parasitic pathogens that might be used include the circumsporozoite (CS) or Liver Stage Specific (LSA) antigens LSA-1 and LSA-3 of *Plasmodium spp.* such as those of P. *bergerii* or *P. falciparum,* or immunogenic mutants thereof; the merozoite surface antigen of *Plasmodium spp.,* the galactose specific lectin of *Entamoeba histolytica,* gp63 of *Leishmania spp.,* gp46 of *Leishmania major,* paramyosin of *Brugia malayi,* the triose-phosphate isomerase of *Schistosoma mansoni,* the secreted globin-like protein of *Trichostrongylus colubriformis,* the glutathione-S-transferase of *Frasciola hepatica, Schistosoma bovis* and *S. japonicum,* and KLH of *Schistosoma bovis* and S. *japonicum.* The viral vectors may also encode synthetic genes, which encode tumor-specific, transplant, or autoimmune antigens or parts thereof. Examples of such antigens include, but are not limited to, prostate specific antigen, MUC1, gp100, HER2, TAG-72, CEA, MAGE-1, tyrosinase, CD3, and IAS beta chain.

The invention also relates to the use of a recombinant adenovirus according to the invention in the preparation of a medicament for the treatment, prophylaxis or diagnosis of an infectious disease. Examples of infectious diseases that are preferably treated, prevented or diagnosed are AIDS, malaria, tuberculosis, ebola virus fever, and hepatitis (A, B and C) infections.

### EXAMPLES

### Example 1: Generation of recombinant Ad35-based viruses having an expression cassette in the E3 region.

Here, the construction of adenoviruses that contain an expression cassette in E1 as well as the E3 region is described. First, a plasmid was made that contains an expression cassette consisting of the human cytomegalovirus (CMV) promoter, a multiple cloning site and the SV40 poly-adenylation signal flanked by BssHII restriction sites. Clearly, the invention is mot limited to the particular promoters, multiple cloning sites and poly-adenylation signals as disclosed herein. The skilled person appreciates that different alternatives exist and may be used. Plasmid pAdApt35 (WO 00/70071) was digested with BssHII, blunted with Klenow enzyme and re-ligated to remove the BssHII site that is present in the multiple cloning site of pAdApt35. After ligation, the expression cassette was amplified by PCR using the following primers: CMV-Bs.F: 5'-CAC AGC GCG CGT CGA CCT AGG TGG TCA ATA TTG GCC -3' (SEQ ID NO:1) and SV40-B.R: 5'-GTG TGC GCG CGA GAT CTA GAC ATG ATA AGA TAC -3' (SEQ ID NO:2).

The reaction was done using Pwo DNA polymerase (Roche) according to manufacturers instructions with the addition of DMSO to a final concentration of 3%. The program was set at 94°C for 2 min followed by 30 cycles of 94°C for 30 sec, 54°C for 30 sec and 72°C for 1.5 min and ended by incubation at 68°C for 8 min. This PCR amplifies the expression cassette and introduces BssHII sites at the ends.

The PCR product was then isolated from agarose gel using the GenecleanII kit (Bio101, Inc.) according to manufacturer's instructions and ligated to the pCR.4-Blunt Topo vector (Invitrogen). This resulted in plasmid pCR-Topo.CMV-sv40. Next, pAdApt35.Luc was digested with HindIII-BamHI and the luciferase gene was isolated from gel using the GenecleanII kit as above. PCR-Topo.CMV-sv40 was digested with the same enzymes and the vector fragment was isolated from gel as above, dephosphorylated with SAP enzyme (Roche) and ligated to the luciferase insert resulting in pCR-Topo.CMV-sv40.Luc. The eGFP isolated from pAdApt35.eGFP was inserted in an analogous manner into pCR-Topo.CMV-sv40 using XbaI restriction enzyme for both the vector and insert. This resulted in pCR.Topo.CMV-sv40-eGFP.

The expression cassettes were then removed from the Topo vectors by digestion with BssHII. This digestion was done in NEB4 buffer together with ApaLI, an enzyme that cuts three times in the Topo backbone fragment, in order to make purification easier. Plasmid pBr.Ad35.PrndE3.50rf6 (WO 03/104467) which contains the 3'part of the Ad35 sequences from the PacI site at nucleotide 18138 (Genbank ref AY271307) to the right ITR with a deletion in the E3 region and the Ad5-derived E4-Orf6 gene replacing the corresponding Ad35 sequences, was linearized with MluI. Both this vector fragment and the Luciferase fragment were purified from gel using the GenecleanII kit. The digested pBr.Ad35.PrndE3.50rf6 vector was then dephosphorylated using SAP enzyme and ligated to the Luc fragment resulting in pBr.Ad35.PRn.dE3-50rf6.CMVsv40.Luc. Plasmid pBr.Ad35.PRn.dE3-50rf6.CMVsv40.eGFP and pBr.Ad35.PRn.dE3-50rf6.CMVsv40 were constructed according to the same procedure starting with a BssHII digestion of pCR.Topo.CMV-sv40-eGFP or pCR.Topo.CMV-sv40 respectively.

Plasmids pBr.Ad35.PRn.dE3-5Orf6.CMVsv40.Luc and pBr.Ad35.PRn.dE3-5Orf6.CMVsv40.eGFP thus contain the 3'part of Ad35 backbone from the PacI site to the right ITR with a CMV-driven Luc or eGFP expression cassette in the E3 region and Ad5-derived E4-Orf6 sequences replacing Ad35 E4-Orf6 sequences.

Recombinant Ad35-based viruses were then generated by co-transfection of an AdApt35Bsu-based adapter plasmid digested with PI-PspI (for a Luc-containing plasmid) or PacI (all others), pWE.Ad35.3481dEcoRV (see below) digested with NotI and EcoRV, and a 3' plasmid based on pBr.Ad35.PRn.dE3-50rf6 digested with PacI and NotI. If plasmids pBr.Ad35.PRn.dE3-50rf6.CMVsv40.Luc and pBr.Ad35.PRn.dE3-50rf6.CMVsv40.eGFP were used DNAs were digested with NotI and PvuI or SwaI and PacI respectively.

Figure 1 schematically shows the different fragments that, following homologous recombination after transfection in to adenovirus packaging cells like PER.C6, form a recombinant Ad35 virus.

Viruses were generated by a combined plaque assay method as follows. 1.5x10⁶ PER.C6^{®} cells/well were seeded in PLL coated 6-well plates the day before transfection, for each virus to be generated one plate is seeded. PER.C6 cells were cultured in DMEM supplemented with 10% FBS (Gibco BRL) and 10 mM MgCl₂. DNAs were digested with appropriate enzymes as described above to liberate the adenoviral inserts from the vector backbone and transfected onto the cells using lipofectamine reagent (Invitrogen) according to manufacturers instructions, using for each transfection (or plate) 360 ng of digested adapter plasmid (left hand fragment), 1440 ng of the pWE.Ad35.3481dEcoRV (middle fragment), and 1080 ng of the pBr.Ad35.PrndE3.50rf6-based plasmid (right hand fragment) supplemented to 25 µl with DMEM (Invitrogen/Gibco BRL). Furthermore 14,4 µl lipofectamine reagent was mixed with DMEM to 25 µl volume. Then DNA and lipofectamine were mixed and incubated 30-40 min at room temperature. Meanwhile medium of the PER.C6 cells was changed for DMEM medium. Just before addition of the DNA to the cells DEM medium was removed from the cells and 0.5 ml DMEM was added to two wells of one plate and 0.25 ml DMEM medium was added to two other wells. The last two wells of each plate did not receive medium. Then 4.5 ml DMEM was added to the incubated DNA-lipofectamine mixture, and after mixing, the mixture was added to the 6-well plate such that all wells contained 1 ml total volume. Plates were incubated at 37°C/10%CO₂ for 4 h. Next, 30 min prior to the end of the incubation time an agar overlay mixture was prepared. Hereto, for each 6-well plate a medium mixture of 9 ml 2xMEM, 0.36 ml FBS, 0.18 ml 1M MgCl₂ and 1.3 ml PBS was made and placed at 37°C. A 2.5% Seaplaque agarose (Cambrex Bio Science) solution was prepared, molten in the microwave and stored at 37°C 15 min before use. At the end of the 4 h incubation period the transfection medium was removed from the cells and the pre-warmed medium and agarose mixtures were removed from the 37°C incubation. 7.5 ml of the agarose mixture was added to the medium mixture with a 10 ml pipet (pre-warmed in a 37°C stove), mixed and added to the cells (3 ml of the mixture/well). The plates were left in the flow cabinet until the overlay was coagulated and then plates were incubated at 37°C/10%CO₂ for 1-2 weeks until plaques appeared. Plaques were picked and expanded on PER.C6 cells from 96-well plate to 6-well plate format. Of each virus five stocks were grown from different plaques and each plaque was serially passaged in 6-well format for 8 passages. Viruses were harvested two days following occurrence of full cytopathogenic effect (CPE) in the cultures, freeze/thawed once and centrifugated for 5 min at 500g in a table top centrifuge. The supernatant was taken and stored at -20°C until further use. For each passage 7,5 µl of the thus prepared crude lysates were used to infect a fresh culture of PER.C6 cells in 6-well plates. At passage number (PN) 2, 5 and 8 DNA was isolated from the viruses and the E1 and E3 regions of the viruses were analyzed by PCR to check integrity using primers that hybridize to sequences flanking the expression cassettes.

The following viruses were generated in this way:
1. Ad35Bsu.dCMV.E3-CMVLuc
2. Ad35Bsu.empty.dE3
3. Ad35Bsu.empty.E3-CMVLuc
4. Ad35Bsu.empty.E3-CMVeGFP
5. Ad35Bsu.eGFP.E3CMVLuc
6. Ad35Bsu.Luc.E3CMVeGFP
7. Ad35Bsu.eGFP.dE3
8. Ad35Bsu.Luc.dE3

Primers used for the PCR over the expression cassette in the former E1 region (E1 PCR) were: ADAPT35CMV-F: 5'-GTA GGT GTC AGC CTA GGT GGT C -3' (SEQ ID NO:3) and Ad35pIX rev N2: 5'-GAT GGG AGA CGC CCT GTC AGA TAA GG -3' (SEQ ID NO:4). Primers used for the expression cassette in the former E3 region (E3 PCR) were: 35E3del: 5'-GTA GCG GTG ATC TCG TAG G -3' (SEQ ID NO:5) and Ad35E3F3: 5'- GTT CAT CTA CTT CGA ACT CC -3' (SEQ ID NO:6).

Amplification was done with recombinant Taq polymerase (Invitrogen) as recommended by the manufacturer. Amplified products were analyzed on agarose gel to detect smaller fragments indicative of deletions in the viral transgene regions. PN4 stocks were used to grow a larger batch of virus (PN5) to enable quantification of the virus by HPLC (Shabram et al. 1997) and *in vitro* expression studies. Hereto, 20x10⁶ (MOI=400) virus particles (VP) of the PN5 crude lysates was used to infect 5x10⁵ A549 cells in a 24-well. Depending on the transgene to be analyzed cells were then harvested for FACS analysis of eGFP expression or for determination of Luciferase activity. The latter was performed with the Steady Glow kit (Promega) according to manufacturers instructions. For measurement of eGFP expression cells were trypsinized, and resuspended in 4 ml PBA buffer (PBS and 0,5% BSA). Following centrifugation cells were then resuspended in 1x Cell-fix (Becton & Dickinson) and analyzed on a FACScalibur (Becton & Dickinson).

PWE.Ad35.3481dEcoRV (WO 2004/001032) is an Ad35-derived fragment containing Ad35 sequences from the pIX coding region to the EcoRV site at nucleotide 24649 in wt Ad35(Genbank ref AY271307).

The plasmid pAdApt35Bsu.dCMV was made as follows. Plasmid pAdApt35Bsu was grown in Inv110 cells, and DNA was isolated and digested with AvrII and XbaI. The un-methylated state of the DNA enabled digestion of an otherwise Dam-methylated XbaI site 3' from the SV40pA sequence. The vector fragment was isolated from gel and relegated resulting in an E1-deleted adapter plasmid without expression cassette.

### Example 2: Stability and expression of double insert viruses.

The viruses prepared as described in example 1 were then analyzed for expression and stability of the transgene regions as described above. Since both cassettes contain the human CMV promoter it was important to analyze whether the expression levels from the E1 and E3 inserts would be comparable (E1 versus E3 expression level) and whether there was any influence of the CMV promoter located in the E3 region on the level of expression from the CMV promoter in the E1 region (promoter interference, also known as 'squelching'). The first question was investigated using an Ad35Bsu.Luc.dE3 virus and an Ad35Bsu.dCMV.E3CMVLuc virus and compare luciferase activity. Figure 2 shows the combined result from 2 independent experiments each performed in triplicate using two different virus preps of each virus. It is clear that the activity of the CMV promoter in the E1 region is approximately 5x higher than that of the CMV promoter in the E3 region. Note that the virus with an insert in the E3 region in this case does not carry a CMV cassette in the E1 region to exclude possible promoter interference. The second question (promoter interference) was investigated with the virus set presented in Figure 3. As can be seen for the luciferase viruses as well as for the eGFP viruses, there is no interference of the presence of an expression cassette in the E3 region on the level of expression from the CMV promoter in the E1 region. This can be concluded from the fact that independent of the presence of an E3 insert (compare virus #2 with #3 and #1 with #4) the level of expression of the E1 insert is the same. The fact that the E3 cassette in virus #4 (E3Luc) and #3 (E3eGFP) shows a lower expression level confirms the above described observation of expression level differences of E1 and E3 inserts.

Next, the stability of the different viruses was analyzed by PCR amplification of the transgene regions. Figure 4 shows the result of the analysis of a double insert virus having a CMV-Luc-SV40pA cassette in the E1 region and a CMV-eGFP-SV40pA cassette in the E3 region. Viruses analyzed at PN2 and PN5 show amplified bands of the correct length for both the E1 and E3 regions. However, the analysis of viruses at PN8 also revealed smaller fragments that likely result from the presence of viruses with deletions or modifications in the transgene regions. The smaller bands that appear in the E1 region PCR could be due to recombination events between the GFP cassette in E3 and the Luc cassette in E1 since the bands were approximately 1 kb less in size. To further investigate this, two more viruses were analyzed, having the CMV-SV40pA empty cassette (no transgene) in the E1 region and either a CMV-Luc-SV40pA or CMV-eGFP-SV40pA in the E3 region. Figure 5 and 6 show the result of the PCR analysis of these viruses. The deletions in the E3 transgene region were now clearly detectable at PN5 and PN8. The bands indicated with 1 and 2 in figure 5 and 6 were isolated, cloned in pCR-Topo vectors and sequenced to reveal the nature of the deletions. The bands indicated with '1' appeared to have an empty expression cassette as was also present in the E1 region. The bands indicated with '2' were deleted for the complete expression cassette. Thus, from the above analysis it is concluded that double insert viruses having the same expression cassettes (but different transgenes) in the E1 and E3 region are not stable for a number of passages of crude virus preps that is comparable with the number of amplification steps that is needed from a plaque purification up to an industrial manufacturing scale.

### Example 3: Increasing stability of double insert viruses by reducing homology in expression cassettes

The viruses that had the type 1 deletions at PN5 shown in Figure 5 and 6 appeared to have acquired the same expression cassette as was present in the E1 region. Since such deletions were never observed when the same Luciferase or eGFP cassettes were present in E1, this was not likely to be due to internal deletion in the E3 cassette and thus suggested that homologous recombination between E1 and E3 caused the deletion in the E3 region. Therefore, it was desired to reduce the homology between the E1 and E3 cassettes. Since the CMV is a very strong promoter that is active in most tissues (and perhaps even the strongest promoter available), it is the preferred promoter for both regions. Therefore, it was investigated whether the recombination would be prevented or reduced by exchanging the poly-adenylation (pA) site only and leaving the CMV promoter in both cassettes. Hereto, new plasmids were generated that contained an E3 cassette consisting of a human CMV promoter and a Bovine Growth Hormone pA (BGHpA) (see below). To first show that the hypothesis that reduction of homology in the pA sequences only indeed leads to increased stability, the BGHpA was initially cloned and tested in the expression cassette in the E1 region. Hereto, the pAdApt35Bsu vector was modified as follows. Plasmid DNA of the pAdApt35Bsu vector isolated from Dam⁻ INV110 cells (Invitrogen) was digested with XbaI and the vector fragment (without the SV40pA sequences) was purified from agarose gel using the GenecleanII kit. The isolated fragment was then dephosphorylated with SAP enzyme. The BGHpA sequence was PCR amplified from plasmid pcDNA-3.1(Neo) (Invitrogen) using the following primers: BGH.pA-XbaI.F: 5'-CAC ATC TAG ACT AGA GCT CGC TGA TCA GCC -3' (SEQ ID NO:7) and BGH.pA-XbaI.R: 5'-GTG TTC TAG AAT CCC CAG CAT GCC TGC TAT -3' (SEQ ID NO:8).

The reaction was performed with Pwo Polymerase using 3% DMSO in the final mixture. The program was set at 94°C for 2 min followed by 30 cycles of 94°C for 30 sec, 62°C for 30 sec and 72°C for 30 sec and ended by incubation at 72°C for 8 min. The 253 basepair (bp) PCR fragment was isolated from gel using the GenecleanII kit. The DNA was then digested with XbaI and purified using the Qiaquick PCR purification kit (Qiagen) according to manufacturers instructions. The above isolated and dephosphorylated vector fragment was then ligated to the isolated and digested BGHpA fragment to give pAdApt35Bsu.BGH. This vector is identical to the empty adapter plasmid pAdApt35Bsu but contains the BGHpA in place of the SV40pA sequences. The luciferase gene, isolated as a BamHI fragment from plasmid pAdApt35Bsu.Luc (WO 2004/001032) was ligated into plasmid pAdApt35Bsu.BGH which was digested with BamHI, isolated from gel and dephosphorylated with SAP enzyme. This gave rise to pAdApt35Bsu.BGH.Luc.

Also the eGFP gene was isolated as an XbaI fragment from pAdApt35Bsu.eGFP (WO 2004/001032) and ligated to pAdApt35Bsu.BGH which had been digested with XbaI (plasmid DNA isolated from DH5α cells), purified from gel and dephosphorylated. This gave rise to pAdApt35Bsu.BGH.eGFP.

Plasmids pAdApt35Bsu and pAdApt35Bsu.BGH were then used to generate recombinant viruses having two expression cassettes as depicted in figure 6 by co-transfection on PER.C6 cells as described in example 1 using also plasmid pWE.Ad35.3481dEcoRV and either plasmid pBr.Ad35.PRn.dE3-50rf6.CMVsv40.Luc or pBr.Ad35.PRn.dE3-50rf6.CMVsv40.eGFP depending on the desired transgene in the E3 region. According to the same procedure as described in example 1, five plaques of each virus type were picked, amplified to 6-well plate format and serially passaged up to PN8. DNA was isolated from the viruses at PN2, PN5 and PN8 to analyze the stability of the expression cassette in the E3 region. Figure 7 and 8 show the result of this direct comparison between Ad35-based viruses with two expression cassettes containing either CMV+SV40 pA in both the E1 and E3 regions or containing an E1 expression cassette with CMV+BGHpA and an E3 cassette consisting of CMV+SV40pA. The viruses with two SV40pA containing expression cassettes show deletion fragments starting at PN5 in 5 out of 5 viruses for both the Luciferase (Figure 7) and the eGFP (Figure 8) cassettes. In contrast, the viruses with the BGHpA in E1 region and SV40pA in the E3 region and having a luciferase gene (Figure 7) were stable at PN5 and showed intact trangene cassettes and no deletion fragments in 3 out of 5 viruses at PN8. Furthermore, the viruses with the BGHpA in E1 region and SV40pA in the E3 region and that had an eGFP cassette (Figure 8) were all stable at PN8. Thus, Ad35-based recombinant viruses having two expression cassettes with the same promoter in which one expression cassette contains a different poly-adenylation sequence compared to the other cassette are stable at least up to PN8. The assays to determine whether such vectors are stable up to PN8 are herewith provided.

### Example 4: Generation of recombinant viruses with two expression cassettes and having different pA sequences.

To generate viruses with the BGHpA in the expression cassette in the E3 region, new plasmids containing the Ad35 right hand sequences were generated. Plasmid PCR.Topo.CMV-SV40.Luc was digested with BamHI and BglII and the ends were made blunt using Klenow enzyme. The vector fragment was isolated from gel using the GenecleanII kit and dephosphorylated using SAP enzyme. The BGHpA was amplified by PCR using pcDNA3.1 (Neo) and digested with XbaI as described in example 3. The protruding ends of the digested PCR fragment were made blunt using Klenow enzyme. The resulting product was purified using the PCR purification kit from Qiagen and isolated from gel. The isolated vector and PCR fragments were then ligated to give pCR.Topo.CMV-BGH.Luc. Care was taken to select a clone in which the pA sequence was in the correct orientation. Then plasmid pBr.Ad35.PrndE3.50rf6 was digested with MluI, isolated from gel and dephosphorylated. Plasmid pCR.Topo.CMV-BGH.Luc was digested with BssHII to isolate the expression cassette. The digestion mixture also contained ApaLI and PvuII to digest the vector fragment in fragments that were easier to separate from the desired insert. The Luciferase expression cassette was then isolated from gel and ligated to the dephosphorylated vector fragment. This gave rise to construct pBr.Ad35.PrndE3.5Orf6.CMV-BGH.Luc. Furthermore, construct pCR.Topo.CMV-SV40.eGFP was digested with XbaI and BglII after which the ends were made blunt using Klenow enzyme. The vector fragment was then isolated from gel and dephosphorylated. This fragment was then ligated to the above isolated blunt BGHpA fragment to give pCR.Topo.CMV-BGH.eGFP.

Plasmid pCR.Topo.CMV-BGH.eGFP is then used to generate pBr.Ad35.PrndE3.50rf6.CMV-BGH.eGFP in the same manner as described above for the luciferase version of this plasmid. With the method described above it is possible to generate also other constructs that are then used to make the stable double insert viruses according to this invention. First, the new transgene is inserted into the pCR.Topo.CMV-BGH.Luc or -eGFP vector by exchanging the transgene sequences making use of convenient matching or blunt restriction enzymes. The then generated shuttle vector is digested with BssHII and the expression cassette containing the gene of interest between a CMV promoter and BGHpA is cloned into the pBr.Ad35.PrndE3.50rf6 vector into the MluI site. The vector that is created in this way is the right hand fragment necessary to reconstitute a recombinant double insert virus as described in example 1. If a transgene contains an internal BssHII site one needs to take special care and perform a partial digest as known in the art in order to isolate the correct intact fragment.

### REFERENCES

Danthinne X (1999) New vectors for the construction of double recombinant adenoviruses. J Virol Meth 81:11-20

Danthinne X (2001) Simultaneous insertion of two expression cassettes into Adenovirus vectors. BioTechniques 30:612-619

Lipinski KS et al. (2001) Tumour-specific adenovirus vectors: repression of transgene expression in healthy cells by endogenous p53. Gene Ther 8:274-281

Martin-Duque P et al. (2004) Direct comparison of the insulating properties of two genetic elements in an adenoviral vector containing two different expression cassettes. Hum Gene Ther 15:995-1002

Shabram PW et al. (1997) Analytical anion-exchange HPLC of recombinant type-5 adenoviral particles. Hum Gene Ther 8:453-465

Tai KF et al. (2003) Concurrent delivery of GM-CSF and endostatin genes by a single adenoviral vector provides a synergistic effect on the treatment of orthotopic liver tumors. J Gene Med 5:386-398

## Claims

1. A recombinant replication-defective adenovirus having an adenoviral genome comprising two separate expression cassettes for the expression of two nucleic acids of interest, said expression cassettes each comprising a promoter and a poly-adenylation signal sequence.

2. A recombinant adenovirus according to claim 1, wherein said adenoviral genome is stable for at least eight passages.

3. A recombinant adenovirus according to claim 1 or 2, wherein said genome comprises a deletion in the E1 region and a deletion in the E3 region.

4. A recombinant adenovirus according to claim 3, wherein one of said two expression cassettes is cloned into the E1 deletion, and the other expression cassette is cloned into the E3 deletion.

5. A recombinant adenovirus according to any one of claims 1-4, wherein said adenovirus is based on an adenovirus serotype selected from Ad11, Ad34, Ad35, Ad48, Ad49 and Ad50.

6. A recombinant adenovirus according to claim 5, wherein said adenovirus is based on Ad35.

7. A recombinant adenovirus according to any one of claims 1-6, wherein said separate expression cassettes comprise the same promoter.

8. A recombinant adenovirus according to claim 7, wherein said promoter is the CMV promoter.

9. A recombinant adenovirus according to any one of claims 1-8, wherein said separate expression cassettes comprise two different poly-adenylation signal sequences.

10. A recombinant adenovirus according to claim 9, wherein one of said poly-adenylation signals is the SV40 pA.

11. A recombinant adenovirus according to claim 9 or 10, wherein one of said poly-adenylation signals is the BGH pA.

12. A recombinant adenovirus according to any one of claims 1-11 as a medicament.

13. The use of a recombinant adenovirus according to any one of claims 1-11 in the preparation of a medicament for the treatment, prophylaxis or diagnosis of an infectious disease.


### Annex to the application documents - subsequently filed sequences listing

SEQUENCE LISTING

<110>  Crucell Holland B.V.
       Vogels, Ronald
       Zuijdgeest, David ATM

<120>  Adenoviral vectors with two separate expression cassettes

<130>  0126 EP P00 PRI

<140>  EP 05110516.1
<141>  2005-11-09

<160>  8

<170>  PatentIn version 3.3

<210>  1
<211>  36
<212>  DNA
<213>  Artificial

<220>
<223>  Synthetic oligonucleotide CMV-Bs.F

<400>  1
cacagcgcgc gtcgacctag gtggtcaata ttggcc                                36


<210>  2
<211>  33
<212>  DNA
<213>  Artificial

<220>
<223>  Synthetic oligonucleotide SV40-B.R

<400>  2
gtgtgcgcgc gagatctaga catgataaga tac                                   33


<210>  3
<211>  22
<212>  DNA
<213>  Artificial

<220>
<223>  Synthetic oligonucleotide ADAPT35CMV-F

<400>  3
gtaggtgtca gcctaggtgg tc                                               22


<210>  4
<211>  26
<212>  DNA
<213>  Artificial

<220>
<223>  Synthetic oligonucleotide Ad35pIX rev N2

<400>  4
gatgggagac gccctgtcag ataagg                                           26


<210>  5
<211>  19
<212>  DNA

<213>  Artificial

<220>
<223>  Synthetic oligonucleotide 35E3del

<400>  5
gtagcggtga tctcgtagg                                                    19


<210>  6
<211>  20
<212>  DNA
<213>  Artificial

<220>
<223>  Synthetic oligonucleotide Ad35E3F3

<400>  6
gttcatctac ttcgaactcc                                                   20


<210>  7
<211>  30
<212>  DNA
<213>  Artificial

<220>
<223>  Synthetic oligonucleotide BGH.pA-XbaI.F

<400>  7
cacatctaga ctagagctcg ctgatcagcc                                        30


<210>  8
<211>  30
<212>  DNA
<213>  Artificial

<220>
<223>  Synthetic oligonucleotide BGH.pA-XbaI.R

<400>  8
gtgttctaga atccccagca tgcctgctat                                        30